(19) **Europäisches Patentamt / European Patent Office / Office européen des brevets**

(11) **EP 2 783 704 A1**

(12) # EUROPEAN PATENT APPLICATION
published in accordance with Art. 153(4) EPC

(43) Date of publication:
**01.10.2014 Bulletin 2014/40**

(21) Application number: **12851918.8**

(22) Date of filing: **22.11.2012**

(51) Int Cl.:
**A61L 15/16** (2006.01)    **A61K 31/734** (2006.01)
**A61K 45/00** (2006.01)    **A61K 47/04** (2006.01)
**A61K 47/12** (2006.01)    **A61K 47/22** (2006.01)
**A61K 47/34** (2006.01)    **A61L 27/00** (2006.01)
**A61P 7/04** (2006.01)

(86) International application number:
**PCT/JP2012/080339**

(87) International publication number:
**WO 2013/077414 (30.05.2013 Gazette 2013/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.11.2011 JP 2011258268**

(71) Applicant: **Otsuka Pharmaceutical Factory, Inc.
Naruto-shi, Tokushima 772-8601 (JP)**

(72) Inventors:
- **FUKUDA, Tatsuru**
  **Naruto-shi**
  **Tokushima 772-8601 (JP)**
- **TAMURA, Hirofumi**
  **Naruto-shi**
  **Tokushima 772-8601 (JP)**

(74) Representative: **HOFFMANN EITLE
Patent- und Rechtsanwälte
Arabellastrasse 4
81925 München (DE)**

(54) **PHARMACEUTICAL COMPOSITION USEFUL FOR ADHESION PREVENTION OR HEMOSTASIS**

(57)    An object of the present invention is to provide a pharmaceutical composition that has excellent water solubility, contains a gelling agent that has a gelation rate in suitable ranges, and is useful as an adhesion preventing biomedical material. Another object of the present invention is to provide a pharmaceutical composition that can demonstrate an excellent hemostatic effect. A solid pharmaceutical composition is prepared from (A) a gelling agent, (B) a salt of a divalent metal and at least one selected from the group consisting of organic acids and inorganic acids, and (C) polyethylene glycol.

EP 2 783 704 A1

**Description**

Technical Field

The present invention relates to a pharmaceutical composition effective as a biomedical material for prevention of adhesion or as a hemostatic agent.

Background Art

Conventionally, gelling agents such as alginates have been used in biomedical materials for use in adhesion prevention, hemostasis, and the like.

Adhesion refers to a state in which organs or tissues that are originally adjacent to, but separate from, each other show unity. Postoperative adhesion at a suture site is a type of artificially generated inflammatory adhesion, and is a complication that is highly likely to be brought about, to various degrees, by an operation. Although adhesion is not problematic when not showing any symptom, adhesion can sometimes cause, for example, stomachache, intestinal obstruction (ileus), and infertility, and therefore, various means have been taken so far to prevent adhesion. Heretofore, reported adhesion preventing biomedical materials that use a gelling agent include a cell blocking membrane containing calcium alginate as a main component (see Patent Literature 1), a composition containing a polysaccharide such as alginic acid, a carboxylic acid group-containing compound, and water (see Patent Literature 2), and the like. When such an adhesion preventing biomedical materials are used (during an operation), the adhesion preventing biomedical material is dissolved in water to allow the gelling agent to gelate, and once a suitable gel strength is reached, the adhesion preventing biomedical material is administered to the affected area.

Such adhesion preventing biomedical materials that use a gelling agent have various gelation rates depending on their compositions, and there are a variety of biomedical materials with which a contact surface promptly undergoes gelation when brought into contact with a gelation accelerator such as calcium and with which gelation takes a considerable amount of time. Adhesion preventing biomedical materials are required to exhibit viscosity that is suitable for application to the affected area when used. An excessively high or excessively low gelation rate of a gelling agent results in poor handleability. Conventional adhesion preventing biomedical materials that use a gelling agent are problematic in that it is difficult to control the gelation rate and their handleability is poor. In particular, if alginic acid is used as a gelling agent, a motor that revolves at a considerable speed is necessary for dissolving alginic acid in water, and thus a dispersion medium such as ethanol is necessary.

On the other hand, as a biomedical material that takes advantage of a hemostatic action of a gelling agent, for example, a wound dressing in a sheet form or sponge form that contains alginate has been reported (see Patent Literature 3). However, with such a gelling agent-containing biomedical material formed into a sheet or sponge, the wound dressing that has been gelated in advance merely covers the surface of the injured site, and does not stop bleeding by undergoing gelation inside the injured site. Therefore, the hemostatic action is not sufficient.

With such conventional art as background, the development of a pharmaceutical composition that has excellent water solubility, contains a gelling agent having a suitable gelation rate, and is effective as an adhesion preventing biomedical material is desired. Moreover, the development of a pharmaceutical composition that has an excellent hemostatic effect due to a gelling agent is desired.

Citation List

Patent Literature

    Patent Literature 1: JP 11-253547A
    Patent Literature 2: JP 2003-153999A
    Patent Literature 3: JP 7-136240A

Summary of Invention

Technical Problem

An object of the present invention is to provide a pharmaceutical composition that has excellent water solubility, contains a gelling agent that has a gelation rate in suitable ranges, and is useful as an adhesion preventing biomedical material. Another obj ect of the present invention is to provide a pharmaceutical composition that can demonstrate an excellent hemostatic effect.

Solution to Problem

As a result of having conducted diligent research to solve the foregoing problems, the inventors found that when a gelling agent, a divalent metal salt of an organic acid and/or inorganic acid, and polyethylene glycol are used in combination, a pharmaceutical composition exhibits excellent water solubility, undergoes gelation at a suitable gelation rate when brought into contact with water, and demonstrates properties suitable for an adhesion preventing biomedical material. Moreover, the inventors found that, when applied in a powder form to an affected part that is accompanied by bleeding, the pharmaceutical composition in which the aforementioned components are used in combination gelates at the affected part and can effectively stop bleeding, and is thus effective as a hemostatic agent. Based on these findings, the inventors conducted further research and accomplished the present invention.
That is, the present invention has the following aspects:

Item 1. A solid pharmaceutical composition containing (A) a gelling agent, (B) a salt of a divalent metal and at least one selected from the group consisting of organic acids and inorganic acids, and (C) polyethylene glycol.
Item 2. The pharmaceutical composition according to Item 1, further containing (D) an organic acid or an alkali metal salt thereof.
Item 3. The pharmaceutical composition according to Item 1 or 2, wherein the ingredient (A) is alginic acid, a pharmaceutically acceptable alginic acid salt, or pectin.
Item 4. The pharmaceutical composition according to any of Items 1 to 3, wherein the divalent metal in the component (B) is calcium.
Item 5. The pharmaceutical composition according to any of Items 1 to 4, wherein the component (B) is at least one selected from the group consisting of dibasic calcium phosphate and calcium gluconate.
Item 6. The pharmaceutical composition according to any of Items 1 to 5, wherein the component (C) has an average molecular weight of about 1000 to about 20000.
Item 7. The pharmaceutical composition according to any of Items 1 to 6, wherein the component (C) is macrogol 4000.
Item 8. The pharmaceutical composition according to any of Items 1 to 7, wherein the component (D) is gluconic acid or glucono-$\delta$-lactone.
Item 9. The pharmaceutical composition according to any of Items 1 to 8, containing 3 to 50 parts by weight of the component (D) relative to 1 part by weight of the component (B).
Item 10. The pharmaceutical composition according to any of Items 1 to 9, used as an adhesion preventing biomedical material.
Item 11. The pharmaceutical composition according to any of Items 1 to 9, used as a hemostatic agent.
Item 12. Use of a solid pharmaceutical composition containing (A) a gelling agent, (B) a salt of a divalent metal and at least one selected from the group consisting of organic acids and inorganic acids, and (C) polyethylene glycol, for the manufacture of an adhesion preventing biomedical material.
Item 13. Use of a solid pharmaceutical composition containing (A) a gelling agent, (B) a salt of a divalent metal and at least one selected from the group consisting of organic acids and inorganic acids, and (C) polyethylene glycol, for the manufacture of a hemostatic agent.
Item 14. An adhesion preventing method including the steps of preparing a solution by mixing an aqueous solvent with a solid pharmaceutical composition containing (A) a gelling agent, (B) a salt of a divalent metal and at least one selected from the group consisting of organic acids and inorganic acids, and (C) polyethylene glycol; and administering the solution to an affected part where adhesion prevention is required.
Item 15. A hemostatic method including the step of administering a solid pharmaceutical composition containing (A) a gelling agent, (B) a salt of a divalent metal and at least one selected from the group consisting of organic acids and inorganic acids, and (C) polyethylene glycol to an affected part where hemostasis is required.

Advantageous Effects of Invention

According to the pharmaceutical composition of the present invention, the gelling agent has enhanced solubility, and a gelation reaction can be started while the pharmaceutical composition is in a uniformly dispersed state in a solvent such as water, thereby making it possible to prepare homogeneous gel without generating clumps. Moreover, when the pharmaceutical composition of the present invention is mixed with a solvent such as water, the gelling agent (hereinafter referred to as the component (A)) and a salt of a divalent metal and an organic acid and/or inorganic acid (hereinafter referred to as the component (B)) are uniformly dispersed in the solvent due to polyethylene glycol (hereinafter referred to as the component (C)), and the divalent metal is gradually released from the component (B), thus enabling gradual gelation. That is, with the pharmaceutical composition of the present invention, adjusting the proportion of the component (A) to the component (B) makes it possible to easily and suitably control the gelation rate. Also, with the pharmaceutical composition of the present invention, adjusting the proportion of the component (A) to the component (B) also makes it possible to control the gel strength so as to be a suitable range. Moreover, gel produced by mixing the pharmaceutical composition of the present invention with a solvent can effectively suppresses adhesion of body tissue such as the tendon, nerve, blood vessel, and organs; cerebral nervous system; and the like. As described above, the pharmaceutical composition of the present invention has excellent water solubility and allows the gelation rate of the gelling agent to be suitably adjusted, and therefore demonstrates excellent handleability in medical settings. Moreover, the pharmaceutical composition demonstrates an excellent adhesion preventing effect on body tissue and is thus effective as an adhesion preventing biomedical material. In particular, the pharmaceutical composition of the present invention is effective as an adhesion preventing biomedical material in the orthopedics field for the tendon, nerve, blood vessel, and the like, and in the digestive surgery field for organs and the like.

In addition, when applied in a powder form to an affected part that is accompanied by bleeding, the pharmaceutical composition of the present invention can demonstrate an excellent hemostatic effect by forming gel at the bleeding site. In particular, the pharmaceutical composition of the present invention can be applied in a powder form as-is and can thus be applied to and around the affected part where hemostasis is required regardless of the shape of the application site, and moreover, gel can form in a state of tightly adhering to the affected part, thus enabling efficient hemostasis.

Moreover, the pharmaceutical composition of the present invention becomes gel on the affected part to which it is applied, and remains on the affected part for a specific period of time. Therefore, the pharmaceutical composition of the present invention if blended with a pharmaceutical agent allows the pharmaceutical agent to be gradually released at the affected part. Also, because the pharmaceutical composition of the present invention can be stored in a solid state, the pharmaceutical composition is thus unlikely to be affected by, for example, temperature, and exhibits excellent storage stability.

Brief Description of Drawings

FIG. 1 is a chart showing the results of measuring the range of motion of the deep digital flexor tendon (difference between bending angles before and after application of load) that is the operative site of each group in Test Example 8.
FIG. 2A is a photograph showing the operative site (deep digital flexor tendon) of a rat of the control group in Test Example 8. FIG. 2B is a photograph showing the operative site (deep digital flexor tendon) of a rat of administration group 1 in Test Example 8.
FIG. 3 is a chart showing the results of evaluating the total adhesion score of each group in Test Example 9.
FIG. 4A is a photograph showing the clamped part of a rat over which the powdery pharmaceutical composition of Example 15 has been spread in Test Example 10. FIG. 4B is a photograph taken when the gelled pharmaceutical composition was removed 5 minutes after spreading the powdery pharmaceutical composition in Test Example 10. FIG. 4C is a photograph taken when sodium alginate power was spread over the clamped part of a rat in Test Example 10.
FIG. 5 is a chart showing the results of measuring the amount of bleeding in each group in Test Example 11.

Description of Embodiments

<Pharmaceutical composition>

The pharmaceutical composition of the present invention is in a solid form and contains (A) a gelling agent, (B) a salt of a divalent metal and at least one selected from the group consisting of organic acids and inorganic acids, and (C) polyethylene glycol. Below, the pharmaceutical composition of the present invention will now be described in detail. Herein, the unit "w/v %" denotes g/100 mL.

Component (A)

The gelling agent (component (A)) used in the present invention is not particularly limited, and is preferably biocompatible or bioabsorbable because it is applied to a living body. Examples include alginic acid, sodium alginate, potassium alginate, propylene glycol alginate, pectin, gellan gum, carrageenan, glucomannan, guar gum, locust bean gum, xanthan gum, glucose, carboxymethyl starch, mannose, galactose, arabinose, fucose, ribose, fructose, dextran, and the like. Preferable examples include alginic acid, sodium alginate, calcium alginate, potassium alginate, propylene glycol alginate, pectin, gellan gum, carrageenan, glucomannan, guar gum, and the like.

From the viewpoint of more effectively imparting an effect to control the gelation rate so as to be within suitable ranges and a hemostatic effect, among these gelling agents, alginic acid, pharmaceutically acceptable salts (for example, alkali metal salts) of alginic acid, alginic acid derivatives, and pectin are suitable. Specific examples of such suitable gelling agents include alginic acid, sodium alginate, calcium alginate, potassium alginate, propylene glycol alginate, and pectin; more preferably, alginic acid, sodium alginate, calcium alginate, potassium alginate, sodium alginate, and pectin; and particularly preferably, sodium alginate.

Such sodium alginate is commercially available, and examples include Kimica Algin High G-series IL-6G (viscosity of 1 w/v% aqueous solution at 20°C = 50 to 80 mPa·s; weight average molecular weight of about 680,000), I-1G (viscosity of 1 w/v% aqueous solution at 20°C = 100 to 200 mPa·s; weight average molecular weight of about 720,000), I-3G (viscosity of 1 w/v% aqueous solution at 20°C = 300 to 400 mPa·s; weight average molecular weight of about 800,000), and the like; Kimica Algin I-series IL-6 (viscosity of 1 w/v% aqueous solution at 20°C = 50 to 80 mPa·s; weight average molecular weight of about 690,000), I-1 (viscosity of 1 w/v% aqueous solution at 20°C = 80 to 200 mPa·s; weight average molecular weight of about 860,000), I-3 (viscosity of 1 w/v% aqueous solution at 20°C = 300 to 400 mPa·s; weight average molecular weight of about 770,000), I-5 (viscosity of 1 w/v% aqueous solution at 20°C = 500 to 600 mPa·s; weight average molecular weight of about 800,000), I-8 (viscosity of 1 w/v% aqueous solution at 20°C = 800 to 900 mPa·s; weight average molecular weight of about 790,000), IL-1 (viscosity of 1 w/v% aqueous solution at 20°C = about 15 mPa·s; weight average molecular weight of about 260,000), IL-2 (viscosity of 1 w/v% aqueous solution at 20°C = 20 to 50 mPa·s; weight average molecular weight of about 580,000), and the like; Kimica Algin ULV-series ULV-5 (viscosity of 10 w/v% aqueous solution at 20°C = 500 to 600 mPa·s; viscosity of 1 w/v% aqueous solution at 20°C = about 4 mPa·s a; weight average molecular weight of about 80,000), ULV-10 (viscosity of 1 w/v% aqueous solution at 20°C = about 7 mPa·s; weight average molecular weight of about 90,000), ULV-20 (viscosity of 1 w/v% aqueous solution at 20°C = about 10 mPa·s; weight average molecular weight of about 200, 000), and the like (all manufactured by Kimica Corporation). Preferable are I-1G, I-3G, I-1, IL-1, ULV-5, ULV-10, ULV-20, and the like.

These component (A) ingredients may be used singly or may be used as a combination of two or more.

When the weight average molecular weight of the gelling agent used as the component (A) is excessively high, the pharmaceutical composition of the present invention demonstrates a high viscosity and may not reach throughout the application site and, in addition, the pharmaceutical composition of the present invention may remain longer than the time needed for adhesion prevention or hemostasis. On the other hand, when the weight average molecular weight of the gelling agent used as the component (A) is excessively low, the pharmaceutical composition is unlikely to stay on the affected part. Accordingly, the weight average molecular weight of the gelling agent may be suitably set in consideration of the use, the application site, and the like of the pharmaceutical composition of the present invention.

For example, in the case where sodium alginate is used as a gelling agent and the pharmaceutical composition of the present invention is used as an adhesion preventing agent, in order to impart suitable retentivity on the affected part, it is preferable to use sodium alginate having a weight average molecular weight of 600000 or less, and in order to impart gel strength required for an adhesion preventing effect, it is preferable to use sodium alginate having a weight average molecular weight of 50000 or greater. In the case where sodium alginate is used as a gelling agent and the pharmaceutical composition of the present invention is used as an adhesion preventing agent, more preferably the weight average molecular weight of sodium alginate may be 80000 to 500000.

In the case where sodium alginate is used as a gelling agent and the pharmaceutical composition of the present invention is used as a hemostatic agent, in order to impart viscosity required for a hemostatic effect, it is preferable to use sodium alginate having a weight average molecular weight of 100000 or greater, and more preferably sodium alginate having a weight average molecular weight of 100,000 to 900,000.

Note that, in the case where two or more types of sodium alginate are used in combination, the aforementioned weight average molecular weight of sodium alginate refers to a value calculated from a mixture of the two or more types of sodium alginate.

The component (A) content of the pharmaceutical composition of the present invention may be, for example, 1 to 99 wt% relative to the total weight of the pharmaceutical composition. More specifically, in the case where the pharmaceutical composition is used as an adhesion preventing agent, from the viewpoint of the dispersibility of the component (A) throughout the pharmaceutical composition and the solubility thereof in a solvent, the content may be preferably 1 to 50 wt% and more preferably 5 to 25 wt%. In the case where the pharmaceutical composition is used as a hemostatic agent, the content may be preferably 40 to 99 wt% and more preferably 50 to 80 wt%.

Component (B)

The salt of a divalent metal and an organic acid and/or inorganic acid used in the present invention (component (B)) is not particularly limited as long as it is pharmaceutically acceptable.

Examples of the acids that constitute the component (B) include organic acids such as gluconic acid, lactic acid, oxalic acid, citric acid, and acetic acid; and inorganic acids such as sulfuric acid, hydrochloric acid, phosphoric acid, and nitric acid.

Examples of the divalent metal that constitutes the component (B) include barium, magnesium, calcium, iron, and the like. Among these divalent metals, calcium is preferable.

Specific examples of the component (B) include calcium lactate, calcium gluconate, calcium sulfate, calcium citrate, dibasic calcium phosphate (calcium monohydrogen phosphate dihydrate), and the like. Among these components (B), from the viewpoint of more effectively imparting an effect to control the gelation rate so as to be within suitable ranges and a hemostatic effect, salts having low water solubility are suitable. Examples of salts having low water solubility include calcium sulfate, calcium citrate, calcium monohydrogen phosphate dihydrate, and calcium carbonate, and homogeneous gel can be produced using an ionization accelerator or retardant. Herein, the term "low water solubility" covers the phrases "slightly soluble", "very slightly soluble", and "practically insoluble or insoluble" defined in The Japanese Pharmacopoeia 16th Edition. That is, the term means that 100 mL or more solvent is needed to dissolve 1 g of solute (component (B)).

Suitable examples of the component (B) include dibasic calcium phosphate, calcium monohydrogen phosphate dihydrate, and calcium carbonate.

These component (B) ingredients may be used singly or may be used as a combination of two or more.

The component (B) content of the pharmaceutical composition of the present invention is, for example, 0.1 to 50 wt% relative to the total weight of the pharmaceutical composition, preferably 1 to 20 wt%, and more preferably 1 to 10 wt%.

Regarding the pharmaceutical composition of the present invention, the proportion of the component (A) to the component (B) is not particularly limited. The higher the proportion of the component (B) to the component (A), the higher the gelation rate and the higher the gel strength. Therefore, in consideration of these behaviors, the proportion is suitably set according to the use, the application site, and the like of the pharmaceutical composition. For example, in the case where the pharmaceutical composition is used as an adhesion preventing agent, satisfying the proportion that no more than 1 part by weight of the component (B) is used per part by weight of the component (A) makes it possible to maintain low gel strength from mixing with an aqueous solvent until administration.

From the viewpoint of more effectively imparting an effect to control the gelation rate so as to be within suitable ranges, an adhesion preventing effect, and a hemostatic effect, it is desirable to satisfy that 0.01 to 1 part by weight, preferably 0.02 to 1 part by weight, and more preferably 0.03 to 0.3 parts by weight of the component (B) is used per part by weight of the component (A).

Component (C)

Polyethylene glycol (PEG) (component (C)) used in the present invention is not particularly limited as long as it is pharmaceutically acceptable.

It is desirable that polyethylene glycol used in the present invention is solid at ordinary temperatures, and the average molecular weight thereof may be, for example, about 1000 or greater and preferably about 3000 or greater. Specifically, the average molecular weight may be about 1000 to about 20000 and preferably about 4000 to about 20000. Polyethylene glycol that has an average molecular weight of less than 1000 is not solid at ordinary temperatures, and polyethylene glycol that has an average molecular weight exceeding 20000 has large viscosity, thus making it difficult to handle such polyethylene glycol during production. Here, the average molecular weight of polyethylene glycol refers to a value measured according to the average molecular weight method for "macrogol 400" of The Japanese Pharmacopoeia 16th Edition.

Specific examples of the component (C) include macrogol 1000, macrogol 1500, macrogol 1540, macrogol 3000, macrogol 3350, macrogol 4000, macrogol 6000, macrogol 8000, macrogol 20000, and the like that are referred to in The Japanese Pharmacopoeia 16th Edition and referred to as pharmaceutical raw materials in Japanese Pharmaceutical Excipients. Among these, from the viewpoint of more effectively imparting an effect to control the gelation rate so as to be within suitable ranges and a hemostatic effect, macrogol 3350, macrogol 4000, macrogol 6000, and macrogol 20000 are preferable, and macrogol 3350 and macrogol 4000 are more preferable.

These component (C) ingredients may be used singly or may be used as a combination of two or more.

From the viewpoint of efficiently dispersing/dissolving other components (component (A) and component (B)) and allowing to react uniformly as well as from the viewpoint of more effectively imparting an effect to control the gelation rate so as to be within suitable ranges and a hemostatic effect by taking advantage of the dissolution rate of polyethylene glycol itself, it is desirable that the component (C) is contained in the pharmaceutical composition of the present invention in a form coating at least the component (A).

The component (C) content of the pharmaceutical composition of the present invention varies depending on the use of the pharmaceutical composition, and may be suitably set usually at a range of 1 to 99 wt% relative to the total weight of the pharmaceutical composition. More specifically, in the case where the pharmaceutical composition of the present invention is used as an adhesion preventing biomedical material, the component (C) content is preferably 20 to 99 wt% and more preferably 50 to 80 wt% relative to the total weight of the pharmaceutical composition. Also, in the case where the pharmaceutical composition of the present invention is used as a hemostatic agent, from the viewpoint of simultaneously achieving blood absorptivity and retentivity on the affected part to more effectively impart a hemostatic effect, the component (C) content is preferably 1 to 20 wt% and more preferably 1 to 10 wt% relative to the total weight of the pharmaceutical composition.

Component (D)

Moreover, the pharmaceutical composition of the present invention may contain, in addition to the components (A) to (C), an organic acid or an alkali metal salt thereof (component (D)). Containing the component (D) makes it possible to further enhance the effect to control the gelation rate so as to be within suitable ranges and the hemostatic effect.

The organic acid used in the present invention is not particularly limited, and examples include glucono-$\delta$-lactone, gluconic acid, glucuronic acid, galacturonic acid, oxalic acid, citric acid, acetic acid, and the like. Examples of the organic acid salt include sodium salts, potassium salts, and the like. Among these, from the viewpoint of more effectively imparting an effect to adjust the gelation rate so as to be within suitable ranges and a hemostatic effect to the pharmaceutical composition of the present invention, glucono-$\delta$-lactone, gluconic acid, and alkali metal salts of gluconic acid are preferable, and sodium gluconate and glucono-$\delta$-lactone are more preferable. Note that glucono-$\delta$-lactone is a compound that is hydrolyzed to gluconic acid when brought into contact with water and shows acidity.

These component (D) ingredients may be used singly or may be used as a combination of two or more.

The component (D) content of the pharmaceutical composition of the present invention may be, for example, 1 to 60 wt% and preferably 2 to 50 wt% relative to the total weight of the pharmaceutical composition. More specifically, in the case where the pharmaceutical composition is used as an adhesion preventing agent, the component (D) content may be 3 to 15 wt%, and in the case where the pharmaceutical composition is used as a hemostatic agent, the component (D) content may be 15 to 40 wt%.

The proportion of blending the component (B) and the component (D) in the present invention is not particularly limited, and from the viewpoint of further enhancing the effect to adjust the gelation rate so as to be within to suitable ranges, the adhesion preventing effect, and the hemostatic effect, it is desirable to satisfy that 0.01 to 80 parts by weight, preferably 2 to 50 parts by weight, and more preferably 3 to 30 parts by weight of the component (D) is used relative to 1 part by weight of the component (B). When the component (D) is less than 0.01 parts by weight relative to 1 parts by weight of the component (B), the gelation rate is low, and it is unlikely that the adhesion preventing effect and the hemostatic effect are demonstrated effectively, and when the component (D) exceeds 80 parts by weight, the gelation rate is excessively high, and handleability is likely to be impaired.

Other components

The pharmaceutical composition of the present invention may contain, in addition to the aforementioned components, pharmacological components such as bactericidal agents, antibiotics, anti-inflammatory agents, blood circulation improving agents, steroids, enzyme inhibitors, growth factors, and various vitamins as necessary in order to, for example, promote the therapeutic effect and prevent bacterial infections. Because the pharmaceutical composition of the present invention gelates on the affected part where the composition is applied and remains for a specific period of time, containing the aforementioned pharmacological components makes it possible to use the pharmaceutical composition of the present invention as a type of drug delivery system intended to gradually release the pharmacological components.

Moreover, the pharmaceutical composition of the present invention may contain additives such as excipients, binders, lubricants, pH adjusters, buffers, preservatives, antioxidants, coloring agents, and desiccants as necessary.

Gelation rate

The components (A) to (C) and, as necessary, the component (D) are contained in the pharmaceutical composition of the present invention, thus making it possible to adjust the gelation rate so as to be within suitable ranges and also impart an excellent hemostatic effect. Here, the suitable gelation rate refers to a rate that can ensure a sufficient time until the pharmaceutical composition is applied to the affected part, and for example, under 25°C, $\omega$ = 3.142 rad/s, and f = 0.5000 Hz conditions in viscoelasticity measurement, the time until reaching a storage elastic modulus G' = a loss rigidity modulus G" may be 1 to 60 minutes, and preferably 3 to 40 minutes. Measurements of the gelation rate can be carried out using a rheometer.

Form

The form of the pharmaceutical composition of the present invention is not particularly limited as long as the pharmaceutical composition is solid, and the pharmaceutical composition is preferably in a powder form. In the case where the pharmaceutical composition of the present invention is in a powder form, the particle size thereof is not particularly limited, and for example, the particle size measured by a sieving method may be about 200 to 2000 $\mu$m and preferably about 355 to 1000 $\mu$m.

Preparation method

The pharmaceutical composition of the present invention is prepared by mixing the components (A) to (C) and, as necessary, the component (D) and other pharmacological components and additives and processing the mixture into a desired form. A suitable example of the preparation method of the pharmaceutical composition of the present invention may include the following steps:

First step of dissolving the component (C);
Second step of admixing the component (A) and the component (B) and, as necessary, the component (D) and other pharmacological components and additives to the solution of the component (C) obtained in the first step; and
Third step of solidifying the mixture obtained in the second step and shaping it into a desired form.

The dissolution of the component (C) in the first step can be carried out by, for example, a method in which the component (C) is thermally dissolved or a method in which the component (C) is dissolved in a solvent. In the case where the component (C) is thermally dissolved, the temperature conditions are suitably set according to the type of the component (C) used, and for example, the temperature may be 50 to 90°C and preferably 60 to 80°C. In the case where the component (C) is dissolved in a solvent, for example, the component (C) may be mixed so as to attain about 5 to 20 wt% with a solvent such as 90 to 99 vol% aqueous ethanol. In the case where the pharmaceutical composition of the present invention is to have a relatively high component (C) content (for example, in the case where the pharmaceutical composition is to be used as an adhesion preventing biomedical material), the first step is preferably carried out by thermal dissolution, and in the case where the pharmaceutical composition of the present invention is to have a relatively low component (C) content (for example, when the pharmaceutical composition is to be used as a hemostatic agent), the first step is preferably carried out by dissolution in a solvent.

In the case where the component (C) is dissolved in a solvent in the first step, the solvent is removed during or after the mixing in the second step. Shaping the mixture into a desired form in the third step can be carried out with a known shaping method such as pulverization or granulation according to the intended shape.

Because the pharmaceutical composition of the present invention is applied to a living body, it is desirable to subject the pharmaceutical composition to a sterilization treatment. The sterilizing method is not particularly limited, and examples include EOG sterilization, electron beam sterilization, $\gamma$ ray sterilization, UV irradiation, and the like, and from the viewpoint of retaining the stability of the gelling agent, electron beam sterilization, EOG sterilization, and $\gamma$ ray sterilization are preferable.

Use

The pharmaceutical composition of the present invention can be used as an adhesion preventing biomedical material or a hemostatic agent.

In the case where the pharmaceutical composition of the present invention is used as an adhesion preventing biomedical material, the pharmaceutical composition is mixed with a suitable amount of aqueous solvent (such as water or physiological saline) to prepare a solution, and when the mixture exhibits a suitable viscosity, the mixture is administered to the affected part where adhesion prevention is required. Once administered to the affected part where adhesion prevention is required, the pharmaceutical composition of the present invention undergoes gelation on the affected part, and gel with a suitable strength that demonstrates an adhesion preventing function is formed. When a solution of the pharmaceutical composition of the present invention is prepared, the proportion of the aqueous solvent mixed is not particularly limited. For example, the aqueous solvent may be set at about 1 to 99 parts by weight and preferably about 3 to 90 parts by weight per part by weight of the pharmaceutical composition of the present invention. Also, a method for administering a solution of the pharmaceutical composition of the present invention to the affected part is not particularly limited, and the solution may be applied to the affected part using, for example, a syringe, brush, or the like. In the case where the pharmaceutical composition of the prevent invention is used as an adhesion preventing biomedical material, the pharmaceutical composition may be administered in an amount that is suitably set according to the condition of the affected part. For example, gel prepared from the pharmaceutical composition of the present invention may be administered in an amount in the range of about 0.005 to 0.1 g per cm$^2$ of the affected part where adhesion prevention is required. In the case where the pharmaceutical composition is used as an adhesion preventing biomedical material, a site to which the pharmaceutical composition is applied is not particularly limited. The pharmaceutical composition is suitably used in the field of surgery on the intra-abdominal organs and the like and in the field of orthopedic surgery on the tendon, nerve, and joint.

In the case where the pharmaceutical composition of the present invention is used as a hemostatic agent, a solution in which the pharmaceutical composition is mixed with a suitable amount of water may be applied to the affected part where hemostasis is required or a paste that is formed by gelating the pharmaceutical composition may be applied to the affected part where hemostasis is required, and it is desirable to apply the pharmaceutical composition that is in a solid form as-is to the affected part where hemostasis is required. The pharmaceutical composition of the present invention when administered to the affected part undergoes gelation on the affected part and forms gel with a suitable strength that demonstrates a hemostatic function. Also, in the case where the pharmaceutical composition of the present invention that in a solid form is administered as-is, an aqueous solvent such as water or physiological saline may be spread over the pharmaceutical composition as necessary after the pharmaceutical composition is administered in order to facilitate the gelation of the pharmaceutical composition. A method for administering the pharmaceutical composition of the present invention that is in a solid form to the affected part is not particularly limited, and for example, the pharmaceutical composition of the present invention that has been granulated by spray drying or the like may be administered to the affected part by spreading or the like. In the case where the pharmaceutical composition of the prevent invention is used as a hemostatic agent, the pharmaceutical composition may be administered in an amount that is suitably set according to the condition of the affected part. For example, gel prepared from the pharmaceutical composition of the present invention may be administered in an amount in the range of about 0.01 to 0.1 g per cm$^2$ of the affected part where hemostatis is required.

Examples

The present invention shall be described in detail below by way of examples, but the present invention is not limited to the examples.

Test Example 1. Evaluation 1: Gelation rate of powdery pharmaceutical composition 1

Preparation

Sodium alginate (types are indicated in Table 1, all manufactured by Kimica Corporation), calcium hydrogenphosphate dihydrate (manufactured by Wako Pure Chemical Industries, Ltd.), and glucono-δ-lactone (manufactured by Spectrum Chemical Mfg. Corp., USA) were added to macrogol 4000 (manufactured by Sanyo Chemical Industries, Ltd.) that had been dissolved at about 70°C, then sufficiently mixed using a stirring bar, and cooled naturally. Thereafter, the mixture was pulverized, and sifted with a No. 22 sieve defined by The Japanese Pharmacopoeia (a sieve mesh of 710 μm), thus giving a powdery pharmaceutical composition passed through the sieve. The amount of each component is as shown in Table 1 below.

Gelation rate measurement

The gelation rate of the powdery pharmaceutical composition obtained above was measured. Specifically, 0.3 g of the powdery pharmaceutical composition was added to 3 mL of purified water and stirred for 30 sec, and 1 min after preparation, the gelation time was measured with a viscosity/viscoelasticity measuring apparatus (rheometer) (HAAKE MARS III: manufactured by Thermo Fisher Scientific Inc.) under 25°C conditions. Specific measurement conditions were as follows.

Temperature control unit: Peltier plate

> Measurement geometry: Parallel plate with 35 mm diameter Gap: 1 mm
> Sample amount: 1 mL
> Applied stress: 11.90 Pa
> Frequency: 0.5000 Hz
> Angular velocity: 3.142 rad/s

The point at which a storage elastic modulus G' and a loss rigidity modulus G" overlapped (crossover point) was regarded as a gel point, and the time until the gel point was reached was regarded as a gel time. Results are also shown in Table 1 below.

Table 1

| | | Ex. 1 | Ex. 2 | Ex. 3 | Ex. 4 | Ex. 5 | Ex. 6 | Ex. 7 | Ex. 8 |
|---|---|---|---|---|---|---|---|---|---|
| Component | Na alginate (upper row: type, lower row: content) | I-1G | I-1G | I-1G | I-1G | I-1G | I-1 | I-3G | I-8 |
| | | 5 | 10 | 15 | 20 | 25 | 5 | 5 | 5 |
| | Macrogol 4000 | 90 | 80 | 70 | 60 | 50 | 90 | 90 | 90 |
| | Calcium hydrogenphosphate dihydrate | 2.5 | 5 | 7.5 | 10 | 12.5 | 2.5 | 2.5 | 2.5 |
| | Glucono-δ-lactone | 2.5 | 5 | 7.5 | 10 | 12.5 | 2.5 | 2.5 | 2.5 |
| Gelation time (min) | | 6.475 | 5.341 | 2.882 | 2.012 | <1 | >10 | 4.066 | 4.631 |

Note 1: The unit of the content of each component is "wt%".

Note 2: In the table, Na alginate is as follows: I-1G denotes Kimica Algin High G-series I-1G having a viscosity of 100 to 200 mPa·s and weight average molecular weight of about 680,000, I-1 denotes Kimica Algin I-series I-1 having a viscosity of 80 to 200 mPa·s and weight average molecular weight of about 860,000, I-3G denotes Kimica Algin High G-series I-3G having a viscosity of 300 to 400 mPa·s and weight average molecular weight of about 800,000, and I-8 denotes Kimica Algin I-series I-8 having a viscosity of 800 to 900 mPa·s and weight average molecular weight of about 790,000. Here, viscosity refers to the viscosity of a 1 w/v% aqueous solution at 20°C.

It was confirmed that in Examples 1 to 8, the solubility in water was enhanced, thus making it possible to form homogeneous gel without generating clumps. On the other hand, a test carried out in the same manner using a pharmaceutical composition having the same composition as Example 1 except that macrogol 4000 was not blended showed that the pharmaceutical composition did not disperse in a uniform manner, forming clumps when the pharmaceutical composition was dissolved in water, and it was difficult to prepare homogeneous gel. Also, a test carried out in the same manner using a pharmaceutical composition having the same composition as Example 1 except that calcium hydrogenphosphate dihydrate was not blended showed that it was not possible to impart sufficient gel strength.

It was also confirmed from the results of Examples 1 to 5 that the greater the macrogol 4000 content, the shorter the gelation time. Moreover, it was confirmed from the results of Examples 6 to 8 that the greater the viscosity of sodium alginate, the shorter the gelation time. Although the measured values of the gel strengths of the pharmaceutical compositions of Examples 5 to 8 are not shown, the gel strengths of the pharmaceutical compositions of Examples 1 to 8 (in particular, Examples 2 to 8) were in a range favorable to demonstrate an adhesion preventing effect.

It is clear from the results provided above that combining sodium alginate (a gelling agent), calcium hydrogenphosphate dihydrate (a salt of an acid and a divalent metal), and macrogol 4000 (polyethylene glycol) enhances the solubility of sodium alginate (a gelling agent) in water, thus allowing homogeneous gel to be prepared without generating clumps. Also, it is clear that suitably configuring the proportions of sodium alginate (a gelling agent), calcium hydrogenphosphate dihydrate (a salt of an acid and a divalent metal), and macrogol 4000 (polyethylene glycol) makes it possible to achieve a desired gelation rate. From these results, it was confirmed that the pharmaceutical compositions of Examples 1 to 8 had properties suitable as adhesion preventing biomedical materials.

Test Example 2. Evaluation 2: Gelation rate of powdery pharmaceutical composition

Preparation

Sodium alginate (Kimica Algin I-series IL-6, viscosity of 1 w/v% aqueous solution at 20°C = 50 to 80 mPa·s, weight average molecular weight of about 690,000, manufactured by Kimica Corporation), calcium hydrogenphosphate (manufactured by Wako Pure Chemical Industries, Ltd.), and glucono-δ-lactone (manufactured by Spectrum Chemical Mfg. Corp. , USA) were added to macrogol 4000 (manufactured by Sanyo Chemical Industries, Ltd.) that had been dissolved at about 70°C, then sufficiently mixed using a stirring bar, and cooled naturally. Thereafter, the mixture was pulverized, and sifted with a No. 30 sieve defined by The Japanese Pharmacopoeia (a sieve mesh of 500 μm), thus giving a powdery pharmaceutical composition passed through the sieve. The amount of each component is as shown in Table 2 below.

Gelation rate measurement

The gelation rate of the powdery pharmaceutical composition obtained above was measured. Specifically, 0.25 g of the powdery pharmaceutical composition was added to 5 mL of purified water and stirred for 10 sec, and 1 min after preparation, the gelation time was measured with a viscosity/viscoelasticity measuring apparatus (rheometer) (HAAKE MARS III: manufactured by Thermo Fisher Scientific Inc.) under 37°C conditions. Specific measurement conditions were as follows.

Temperature control unit: Peltier plate
Measurement geometry: Parallel plate with 35 mm diameter Gap: 1 mm
Sample amount: 1 mL
Applied stress: 1 Pa
Frequency: 0.5000 Hz
Angular velocity: 3.142 rad/s

The point at which G' and G" overlapped (crossover point) was regarded as a gel point, and the time until the gel point was reached was regarded as a gel time. The values of the storage elastic modulus (G') and the loss rigidity modulus (G") at the crossover point, the gelation time, the viscosity at the crossover point ($|\eta^*|$) are also shown in Table 2.

Table 2

| | | Example | | | | | | |
|---|---|---|---|---|---|---|---|---|
| | | 9 | 10 | 11 | 12 | 13 | 14 | 15 |
| Component | Na alginate | 5 | 10 | 15 | 20 | 25 | 30 | 50 |
| | Ca hydrogenphosphate | 1 | 2 | 3 | 4 | 5 | 6 | 10 |
| | Glucono-δ-lactone | 3 | 6 | 9 | 12 | 15 | 18 | 30 |
| | Macrogol 4000 | 91 | 82 | 73 | 64 | 55 | 46 | 10 |
| Gelation time (min) | | 84.89 | 72.26 | 38.58 | 25.12 | 16.68 | 12.91 | 7.25 |
| G',G" (Pa) at crossover point | | 0.195 | 0.865 | 1.228 | 2.356 | 4.175 | 4.195 | 12.53 |
| Viscosity \|η*\| (mPa·s) at crossover point | | 88 | 374 | 578 | 1038 | 1972 | 1906 | 5970 |

Note: The unit of the content of each component is "wt%".

It was confirmed from the results presented above that the larger the sodium alginate content, the shorter the gelation time and the higher the viscosity. That is, it is clear that adjusting the sodium alginate concentration makes it possible to control the gelation time and the gel strength attained after the pharmaceutical composition is brought into contact with water.

Test Example 3. Evaluation 3: Gelation rate of powdery pharmaceutical composition

Preparation

Sodium alginate (Kimica Algin I-series IL-1, viscosity of 1 w/v% aqueous solution at 20°C = about 15 mPa·s, weight average molecular weight of about 260,000, manufactured by Kimica Corporation), calcium hydrogenphosphate (manufactured by Wako Pure Chemical Industries, Ltd.), and glucono-δ-lactone (manufactured by Spectrum Chemical Mfg. Corp., USA) were added to macrogol 4000 (manufactured by Sanyo Chemical Industries, Ltd.) that had been dissolved at about 70°C, then sufficiently mixed using a stirring bar, and cooled naturally. Thereafter, the mixture was pulverized, and sifted with a No. 30 sieve defined by The Japanese Pharmacopoeia (a sieve mesh of 500 μm), thus giving a powdery pharmaceutical composition passed through the sieve. The amount of each component is as shown in Table 3 below.

Gelation rate measurement

The gelation rate of the powdery pharmaceutical composition obtained above was measured. Specifically, the powdery pharmaceutical composition was added to purified water so as to attain the concentrations shown in Table 3 and then stirred for 10 sec, and 1 min after preparation, the gelation time was measured with a viscosity/viscoelasticity measuring apparatus (rheometer) (HAAKE MARS III: manufactured by Thermo Fisher Scientific Inc.) under 37°C conditions. Specific measurement conditions are the same as in Test Example 2. The obtained results are shown in Table 3.

Table 3

| Component | | Ex. 16 | Ex. 17 | Ex. 18 | Ex. 19 | Ex. 20 | Ex. 21 | Ex. 22 | Ex. 23 | Ex. 24 | Ex. 25 | Ex. 26 | Ex. 27 |
|---|---|---|---|---|---|---|---|---|---|---|---|---|---|
| | Na alginate | 20 | 20 | 20 | 20 | 20 | 20 | 20 | 20 | 20 | 20 | 20 | 10 |
| | Macrogol 4000 | 78.8 | 77.6 | 76 | 74 | 72 | 70 | 68 | 64 | 60 | 60 | 40 | 40 |
| | Ca hydrogenphosphate | 0.2 | 0.4 | 0.67 | 1 | 1.3 | 1.7 | 2 | 4 | 6 | 10 | 20 | 40 |
| | Glucono-δ-lactone | 1 | 2 | 3.33 | 5 | 6.7 | 8.3 | 10 | 12 | 14 | 10 | 20 | 10 |
| | Concentration in purified water (g/100 mL) | 20 | 20 | 20 | 10 | 10 | 10 | 10 | 10 | 10 | 10 | 10 | 10 |
| | Gelation time (min) | 130.20 | 74.11 | 51.78 | 78.93 | 64.91 | 37.13 | 25.52 | 12.98 | 7.77 | 8.58 | 4.72 | 3.72 |
| | G',G" (Pa) at crossover point | 4.525 | 5.386 | 5.539 | 1.246 | 0.999 | 0.887 | 1.135 | 1.623 | 1.406 | 1.704 | 1.257 | 0.496 |
| | Viscosity |η*| (mPa·s) at crossover point | 2024 | 2412 | 2510 | 562 | 450 | 402 | 505 | 724 | 619 | 752 | 557 | 222 |

Note: The unit of the content of each component is "wt%".

It was confirmed from the results presented above that the larger the proportion of calcium hydrogenphosphate to sodium alginate, the shorter the gelation time and the higher the viscosity. That is, it is clear that adjusting the proportion of sodium alginate to calcium hydrogenphosphate makes it possible to control the gelation time and the gel strength attained after the pharmaceutical composition is brought into contact with water.

Test Example 4. Evaluation 4: Gelation rate of powdery pharmaceutical composition

Preparation

Sodium alginate (Kimica Algin I-series IL-1, viscosity of 1 w/v% aqueous solution at 20°C = about 15 mPa·s, weight average molecular weight of about 260,000, manufactured by Kimica Corporation), calcium hydrogenphosphate (manufactured by Wako Pure Chemical Industries, Ltd.), and glucono-$\delta$-lactone (manufactured by Spectrum Chemical Mfg. Corp., USA) were added to macrogol 4000 (manufactured by Sanyo Chemical Industries, Ltd.) that had been dissolved at about 70°C, then sufficiently mixed using a stirring bar, and cooled naturally. Thereafter, the mixture was pulverized, and sifted with a No. 30 sieve defined by The Japanese Pharmacopoeia (a sieve mesh of 500 $\mu$m), thus giving a powdery pharmaceutical composition passed through the sieve. The amount of each component is as shown in Table 4 below.

Gelation rate measurement

The gelation rate of the powdery pharmaceutical composition obtained above was measured. Specifically, 1 g of the powdery pharmaceutical composition was added to 10 mL of purified water and stirred for 10 sec, and 1 min after preparation, the gelation time was measured with a viscosity/viscoelasticity measuring apparatus (rheometer) (HAAKE MARS III: manufactured by Thermo Fisher Scientific Inc.) under 37°C conditions. Specific measurement conditions are the same as in Test Example 2. The obtained results are shown in Table 4.

Table 4

| Component | Ex. 28 | Ex. 29 | Ex. 30 | Ex. 31 | Ex. 32 | Ex. 33 | Ex. 34 | Ex. 35 | Ex. 36 | Ex. 37 | Ex. 38 |
|---|---|---|---|---|---|---|---|---|---|---|---|
| Na alginate | 20 | 20 | 20 | 20 | 20 | 20 | 20 | 20 | 20 | 20 | 10 |
| Macrogol 4000 | 69.9 | 69.5 | 69 | 60 | 74 | 69 | 49 | 29 | 19 | 9 | 9 |
| Ca hydrogenphosphate | 10 | 10 | 10 | 10 | 1 | 1 | 1 | 1 | 1 | 1 | 1 |
| Glucono-δ-lactone | 0.1 | 0.5 | 1 | 10 | 5 | 10 | 30 | 50 | 60 | 70 | 80 |
| Ca hydrogenphosphate : glucono-δ-lactone | 1:0.01 | 1:0.05 | 1:0.1 | 1:1 | 1:5 | 1:10 | 1:30 | 1:50 | 1:60 | 1:70 | 1:80 |
| Na alginate : Ca hydrogenphosphate | 1:0.5 | 1:0.5 | 1:0.5 | 1:0.5 | 1:0.05 | 1:0.05 | 1:0.05 | 1:0.05 | 1:0.05 | 1:0.05 | 1:0.1 |
| Gelation time (min) | 158.9 | 48.10 | 40.37 | 35.25 | 63.63 | 12.80 | 10.23 | 7.033 | 5.113 | 3.175 | 2.013 |
| G', G" (Pa) at crossover point | 4.493 | 2.347 | 2.731 | 0.8822 | 1.311 | 1.607 | 1.010 | 0.8837 | 0.9616 | 1.595 | 1.304 |
| Viscosity \|n*\| (mPa·s) at crossover point | 2103 | 1034 | 1254 | 377.6 | 607.7 | 745.9 | 409.1 | 375.5 | 367.8 | 426.7 | 375.8 |

Note: The unit of the content of each component is "wt%".

It was confirmed from the results presented above that the greater the proportion of glucono-δ-lactone to calcium hydrogenphosphate, the shorter the gelation time. That is, it is clear that adjusting the proportion of glucono-δ-lactone to calcium hydrogenphosphate makes it possible to control the gelation time attained after the pharmaceutical composition is brought into contact with water.

Test Example 5. Evaluation 5: Gelation rate of powdery pharmaceutical composition

Preparation

Sodium alginate, calcium hydrogenphosphate (manufactured by Wako Pure Chemical Industries, Ltd.), and glucono-$\delta$-lactone (manufactured by Spectrum Chemical Mfg. Corp., USA) were added to macrogol 4000 (manufactured by Sanyo Chemical Industries, Ltd.) that had been dissolved at about 70°C, then sufficiently mixed using a stirring bar, and cooled naturally. Thereafter, the mixture was pulverized, and sifted with a No. 30 sieve defined by The Japanese Pharmacopoeia (a sieve mesh of 500 $\mu$m), thus giving a powdery pharmaceutical composition passed through the sieve. The amount of each component is as shown in Tables 5 to 9 below. In Tables 5 to 9, the types of Na alginate are as follows.

A: a mixture of 90 parts by weight of Kimica Algin ULV-series ULV-5 (10 w/v% aqueous solution, viscosity of 500 to 600 mPa·s at 20°C, manufactured by Kimica Corporation) and 10 parts by weight of Kimica Algin I-series I-1 (viscosity of 1 w/v% aqueous solution at 20°C = 80 to 200 mPa·s, weight average molecular weight of about 860,000, manufactured by Kimica Corporation),
B: ULV-10 (viscosity of 1 w/v% aqueous solution at 20°C = about 7 mPa·s, weight average molecular weight of about 90,000, manufactured by Kimica Corporation)
C: ULV-20 (viscosity of 1 w/v% aqueous solution at 20°C = about 10 mPa·s, weight average molecular weight of about 200,000, manufactured by Kimica Corporation)
D: Kimica Algin High G-series IL-1 (viscosity of 1 w/v% aqueous solution at 20°C = about 15 mPa·s, weight average molecular weight of about 260,000, manufactured by Kimica Corporation) E: Kimica Algin I-series IL-6 (viscosity of 1 w/v% aqueous solution at 20°C = 50 to 80 mPa·s, weight average molecular weight of about 690,000, manufactured by Kimica Corporation)

Gelation rate measurement

The gelation rate of the powdery pharmaceutical composition obtained above was measured. Specifically, the powdery pharmaceutical composition was added to purified water so as to attain the concentrations shown in Tables 5 to 9 and then stirred for 10 sec, and 1 min after preparation, the gelation time was measured with a viscosity/viscoelasticity measuring apparatus (rheometer) (HAAKE MARS III: manufactured by Thermo Fisher Scientific Inc.) under 37°C conditions. Moreover, the viscosity (|n*|) at the beginning of measurement was also measured. Specific measurement conditions are the same as in Test Example 2. The obtained results are shown in Table 5.

Table 5

| | | | Ex. 28 | | | Ex. 29 | | Ex. 30 | | Ex. 31 | Ex. 32 | Ex. 33 | Ex. 34 |
|---|---|---|---|---|---|---|---|---|---|---|---|---|---|
| Component | Na alginate | Type | A | | | B | | C | | D | D | E | D |
| | | Content | 20 | | | 20 | | 20 | | 20 | 20 | 20 | 20 |
| | Macrogol 4000 | | 68 | | | 68 | | 68 | | 68 | 64 | 64 | 68 |
| | Ca hydrogenphosphate | | 2 | | | 2 | | 2 | | 2 | 4 | 4 | 2 |
| | Glucono-δ-lactone | | 10 | | | 10 | | 10 | | 10 | 12 | 12 | 10 |
| Concentration in purified water(g/100 mL) | | | 5 | 10 | 25 | 10 | 25 | 10 | 25 | 10 | 25 | 10 | 10 |
| Viscosity $|\eta^*|$ (mPa·s) at beginning of measurement | | | 15 | 22 | 160 | 22 | 140 | 28 | 298.4 | 42 | 1482 | 308.8 | 2 |
| Gelation time (min) | | | 69.50 | 31.74 | 7.30 | 43.23 | 8.48 | 35.16 | 7.41 | 15.09 | 1.15 | 2.98 | 25.52 |
| G',G" (Pa) at crossover point | | | 0.374 | 0.890 | 2.669 | 0.991 | 2.870 | 1.113 | 3.798 | 0.872 | 6.655 | 2.765 | 1.135 |
| Viscosity $|\eta^*|$ (mPa·s) at crossover point | | | 168 | 416 | 1212 | 428 | 1249 | 488 | 1653 | 383 | 2907 | 1250 | 505 |

Note: The unit of the content of each component is "wt%".

Table 6

| Component | | | Ex. 35 | | Ex. 36 | | Ex. 37 | | Ex. 38 | |
|---|---|---|---|---|---|---|---|---|---|---|
| | Na alginate | Type | A | | A | | A | | A | |
| | | Content | 20 | | 20 | | 20 | | 20 | |
| | Macrogol 4000 | | 68 | | 70 | | 72 | | 74 | |
| | Ca hydrogenphosphate | | 2 | | 1.7 | | 1.3 | | 1 | |
| | Glucono-δ-lactone | | 10 | | 8.3 | | 6.7 | | 5 | |
| Concentration in purified water (g/100 mL) | | | 7 | 14 | 10 | 14 | 10 | 14 | 10 | 14 |
| Viscosity \|η*\| (mPa·s) at beginning of measurement | | | 15 | 46 | 25 | 44 | 24 | 37.28 | 25 | 42 |
| Gelation time (min) | | | 43.52 | 10.24 | 29.37 | 13.4 | 41.49 | 26.02 | 63.51 | 47.54 |
| G',G" (Pa) at crossover point | | | 0.7153 | 1.306 | 0.8877 | 1.193 | 0.8167 | 1.012 | 0.9244 | 1.021 |
| Viscosity \|η*\| (mPa·s) at crossover point | | | 319.6 | 569 | 403.9 | 549.4 | 366.5 | 452.8 | 415.2 | 458.5 |

Note: The unit of the content of each component is "wt%".

Table 7

| Component | | | Ex. 39 | | Ex. 40 | | Ex. 41 | | Ex. 42 | |
|---|---|---|---|---|---|---|---|---|---|---|
| | Na alginate | Type | B | | B | | B | | B | |
| | | Content | 20 | | 20 | | 20 | | 20 | |
| | Macrogol 4000 | | 68 | | 70 | | 72 | | 74 | |
| | Ca hydrogenphosphate | | 2 | | 1.7 | | 1.3 | | 1 | |
| | Glucono-δ-lactone | | 10 | | 8.3 | | 6.7 | | 5 | |
| Concentration in purified water(g/100 mL) | | | 10 | 14 | 10 | 14 | 10 | 14 | 10 | 14 |
| Viscosity $|\eta*|$ (mPa·s) at beginning of measurement | | | 22 | 37 | 18 | 33 | 18 | 33 | 20 | 34 |
| Gelation time (min) | | | 43.23 | 20.96 | 48.97 | 22.55 | 63.01 | 43.18 | 78.47 | 65.72 |
| G',G" (Pa) at crossover point | | | 0.991 | 1.22 | 0.9263 | 1.023 | 0.755 | 0.9921 | 0.864 | 1.01 |
| Viscosity $|\eta*|$ (mPa·s) at crossover point | | | 427.6 | 547.8 | 416.4 | 460 | 338.2 | 447.1 | 388.6 | 463 |

Note: The unit of the content of each component is "wt%".

EP 2 783 704 A1

Table 8

| | | | Ex. 43 | | Ex. 44 | | Ex. 45 | | Ex. 46 | |
|---|---|---|---|---|---|---|---|---|---|---|
| Component | Na alginate | Type | C | | C | | C | | C | |
| | | Content | 20 | | 20 | | 20 | | 20 | |
| | Macrogol 4000 | | 68 | | 70 | | 72 | | 74 | |
| | Ca hydrogenphosphate | | 2 | | 1.7 | | 1.3 | | 1 | |
| | Glucono-δ-lactone | | 10 | | 8.3 | | 6.7 | | 5 | |
| Concentration in purified water(g/100 mL) | | | 10 | 14 | 10 | 14 | 10 | 14 | 10 | 14 |
| Viscosity $|\eta^*|$ (mPa·s) at beginning of measurement | | | 28 | 43.89 | 19.01 | 45 | 23 | 42 | 25 | 46 |
| Gelation time (min) | | | 35.16 | 23.44 | 48.96 | 27.3 | 59.11 | 37.75 | 76.87 | 60.86 |
| G',G" (Pa) at crossover point | | | 1.113 | 1.205 | 0.9645 | 1.253 | 1.177 | 1.353 | 1.203 | 1.288 |
| Viscosity $|\eta^*|$ (mPa·s) at crossover point | | | 487.6 | 530 | 434 | 557 | 534 | 606.9 | 542.5 | 582.4 |

Note: The unit of the content of each component is "wt%".

Table 9

| Component | | Ex. 47 | | Ex. 48 | | Ex. 49 | | Ex. 50 | |
|---|---|---|---|---|---|---|---|---|---|
| Na alginate | type | D | | D | | D | | D | |
| | content | 20 | | 20 | | 20 | | 20 | |
| Macrogol 4000 | | 68 | | 70 | | 72 | | 74 | |
| Ca hydrogenphosphate | | 2 | | 1.7 | | 1.3 | | 1 | |
| Glucono-δ-lactone | | 10 | | 8.3 | | 6.7 | | 5 | |
| Concentration in purified water (g/100 mL) | | 14 | 10 | 14 | 10 | 14 | 10 | 14 | 10 |
| Viscosity $|\eta^*|$ (mPa·s) at beginning of measurement | | 84 | 42 | – | – | – | – | – | – |
| Gelation time (min) | | 9.358 | 15.09 | – | – | – | – | – | – |
| G', G" (Pa) at crossover point | | 2.395 | 0.872 | – | – | – | – | – | – |
| Viscosity $|\eta^*|$ (mPa·s) at crossover point | | 1075 | 383 | – | – | – | – | – | – |

Note: The unit of the content of each component is "wt%".

Note: "–" denotes "not determined".

The pharmaceutical compositions of Examples 28 to 50 all had gelling rates and gel strengths within suitable ranges and sufficiently satisfied properties required as adhesion preventing biomedical materials.

Test Example 6. Preparation of powdery pharmaceutical composition

Preparation

Sodium alginate (Kimica Algin High G-series I-1G, viscosity of 1 w/v% aqueous solution at 20°C = 100 to 200 mPa·s, weight average molecular weight of about 720, 000, manufactured by Kimica Corporation), calcium hydrogenphosphate dihydrate (manufactured by Wako Pure Chemical Industries, Ltd.), and glucono-δ-lactone (manufactured by Spectrum Chemical Mfg. Corp., USA) were sufficiently mixed with each other, then added to a 5 wt% solution of macrogol 4000 (water was used as a solvent), and kneaded using an agate mortar while being exposed to warm air, thus giving a mixture. Thereafter, the mixture was pulverized, and sifted with a No. 22 sieve defined by The Japanese Pharmacopoeia (a sieve mesh of 710 μm), thus giving a powdery pharmaceutical composition. The amount of each component is as shown in Table 10 below. These powdery pharmaceutical compositions had properties suitable as hemostatic agents.

Table 10

| | Ex. 51 | Ex. 52 | Ex. 53 | Ex. 54 | Ex. 55 | Ex. 56 | Ex. 57 | Ex. 58 | Ex. 59 | Ex. 60 | Ex. 61 | Ex. 62 |
|---|---|---|---|---|---|---|---|---|---|---|---|---|
| Na alginate | 2 | 2 | 2 | 2 | 2 | 2 | 2 | 2 | 2 | 4 | 4 | 4 |
| Macrogol 4000 | 0.25 | 0.5 | 1 | 0.25 | 0.5 | 1 | 0.1 | 0.2 | 0.5 | 0.2 | 0.3 | 0.4 |
| Ca hydrogenphosphate dihydrate | 0.8 | 0.8 | 0.8 | 0.4 | 0.4 | 0.4 | 0.2 | 0.2 | 0.2 | 0.2 | 0.2 | 0.2 |
| Glucono-$\delta$-lactone | 4 | 4 | 4 | 2 | 2 | 2 | 1 | 1 | 1 | 1 | 1 | 1 |
| Total | 7.05 | 7.3 | 7.8 | 4.65 | 4.9 | 5.4 | 3.3 | 3.4 | 3.7 | 5.4 | 5.5 | 5.6 |
| Note: The unit of the content of each component is "g". | | | | | | | | | | | | |

Test Example 7. Evaluation 6: Gelation rate of powdery pharmaceutical composition

A powdery pharmaceutical composition was obtained in the same manner as in Test Example 5 except that pectin (SM-666 and Vistop D-1382, both manufactured by San-Ei Gen F.F.I., Inc.) was used in place of sodium alginate. The amount of each component is as shown in Table 11 below. The gelation rates were measured in the same manner as in Test Example 5 using the resulting powdery pharmaceutical compositions. Results are shown in Table 11 below.

Table 11

| | | | Ex. 63 | Ex. 64 |
|---|---|---|---|---|
| Component | Pectin | Type | SM-666 | Vistop® D-1382 |
| | | Content | 20 | 20 |
| | Calcium hydrogenphosphate | | 2 | 2 |
| | Glucono-$\delta$-lactone | | 10 | 10 |
| | Macrogol 4000 | | 68 | 68 |
| Concentration in purified water(g/100 mL) | | | 10 | 10 |
| Viscosity |n*| (mPa·s) at beginning of measurement | | | 9516 | 24 |
| Gelation time (min) | | | 1.49 | 4.73 |
| G',G" (Pa) at crossover point | | | 3.900 | 3.096 |
| Viscosity |$\eta$*| (mPa·s) at crossover point | | | 1727 | 1339 |
| Note: The unit of the content of each component is "wt%". | | | | |

The pharmaceutical compositions of Examples 63 and 64 both had gelling rates and gel strengths within suitable ranges and sufficiently satisfied properties required as adhesion preventing biomedical materials.

Test Example 8. Adhesion preventing effect 1: Tendon

An adhesion preventing effect on the tendon was evaluated using male rats (Crlj:WI) (obtained from Charles River Laboratories Japan, Inc.). Specifically, first, the synovial tendon sheath in the plantar part of a rat was longitudinally incised, the deep digital flexor tendon was then exposed and cut to a halfway point so as to prepare a tendon-injured model rat. Next, the test substances shown in Table 12 were each administered in 50 $\mu$L amounts to the operative site where the tendon was cut to the halfway point and its surroundings. Then, in order to prevent the tendon that had been cut to the halfway point from rupturing caused by the rat's own movement, the sciatic nerve was cut to limit motility of the rat, and the rat was reared for 4 weeks. After 4 weeks, the foot of each rat was removed at the ankle so as to have only the long flexor muscle. A 60 g load was applied to the long flexor muscle of the removed foot, and the angle of the metatarsophalangeal joint (MTP) and the angle of the proximal interphalangeal joint (PIP) of the second finger before and after the application of the load were measured. According to the expression below, the difference between the bending angles before and after the application of the load was calculated. For comparison, the difference between the bending angles of a normal finger in which the deep digital flexor tendon was not cut to a halfway point was also measured before and after the application of a load.

Expression 1

$$\text{Difference between bending angles before and after application of load (°) = (Angle of MTP after application of load + angle of PIP after application of load) - (Angle of MTP before application of load + angle of PIP before application of load)}$$

Table 12

|  | Test substance |
| --- | --- |
| Control group | Physiological saline |
| Administration group 1 | The pharmaceutical composition of Example 28 was added to physiological saline so as to attain 10 g/100 mL and stirred for 10 seconds, and the solution within 1 minute from preparation was used for administration. |
| Administration group 2 | The pharmaceutical composition of Example 28 was added to purified water so as to attain 25 g/100 mL and stirred for 10 seconds, and the solution within 1 minute from preparation was used for administration. |
| Administration group 3 | The pharmaceutical composition of Example 34 was added to physiological saline so as to attain 10 g/100 mL and stirred for 10 seconds, and the solution within 1 minute from preparation was used for administration. |
| Administration group 4 | The pharmaceutical composition of Example 22 was added to physiological saline so as to attain 10 g/100 mL and stirred for 10 seconds, and the solution within 1 minute from preparation was used for administration. |
| Administration group 5 | The pharmaceutical composition of Example 23 was added to purified water so as to attain 25 g/100 mL and stirred for 10 seconds, and the solution within 1 minute from preparation was used for administration. |

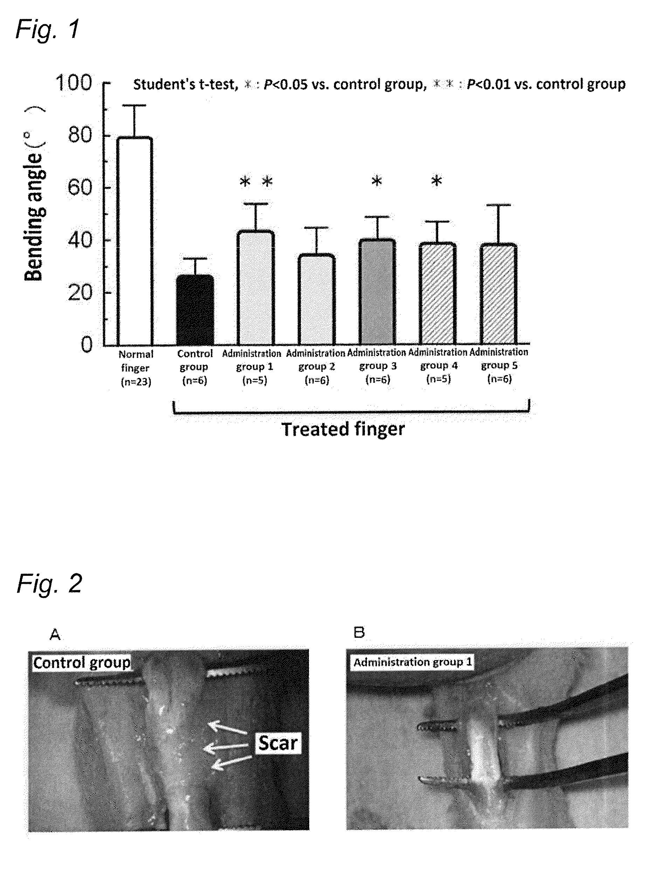

The obtained results are shown in FIG. 1. It was confirmed from the results that administration groups 1 to 5 showed greater differences in values between the bending angles before and after the application of the load than that of the control group, and thus the range of motion of the deep digital flexor tendon at the operative site was increased, and postoperative adhesion was effectively suppressed.

FIG. 2A shows the operative site (deep digital flexor tendon) 4 weeks after the operation on a rat of the control group, and FIG. 2B shows the operative site (deep digital flexor tendon) 4 weeks after the operation on a rat of administration group 1. As is clear from FIG. 2A, in the control group administered with physiological saline, strong adhesion between scar tissue attached to the tendon and surrounding tissue was observed. On the other hand, as is clear from FIG. 2B, in administration group 1 administered with the pharmaceutical composition of Example 28 together with physiological saline, almost no scar tissue was present in the tendon, and adhesion was considerably suppressed.

Test Example 9. Adhesion preventing effect 2: Peritoneum-cecum

A preventing effect on adhesion of the sidewall and the cecum was evaluated using male rats (Crlj:WI) (obtained from Charles River Laboratories Japan, Inc.). Specifically, first, a 1×4 cm piece of a rat's right peritoneal external oblique muscle and internal oblique muscle was excised to prepare a sidewall. Next, the inside of the sidewall was rubbed with gauze. Also, the cecum was removed from the rat, and the entire cecum was rubbed with gauze and then left to stand for 20 minutes at room temperature while being exposed to air. Thereafter, the cecum was washed with a lactated Ringer's solution (manufactured by Otsuka Pharmaceutical Factory, Inc.), and the test substances shown in Table 13 were each administered to the entire area of the cecum. Next, the cecum that had been treated in the above-described manner was attached to the sidewall part and brought back inside the peritoneal cavity, then the abdomen was closed, and the rat was reared for 1 week. Laparotomy was performed 1 week later to observe adhesion of the sidewall and the cecum. Adhesion was evaluated according to the criteria shown below to yield an adhesion site score and an adhesion severity score, and these scores were added up to give a total adhesion score.

<Adhesion site score>

Score: State

0: No adhesion
1: Adhesion observed only in sidewall removed site
2: Adhesion observed in both sidewall removed site and inside of sidewall

<Adhesion severity score>

Score: state

1: Mild adhesion; adhesion easily removable by hand
2: Moderate adhesion; adhesion requiring blunt dissection and removable
3: Severe adhesion; adhesion requiring blunt dissection and not removable without tissue damage

Table 13

|  | Test substance |
|---|---|
| Control group A | None |
| Control group B | Lactated Ringer's solution (manufactured by Otsuka Pharmaceutical Factory, Inc.) |
| Administration group A | The pharmaceutical composition of Example 28 was added to purified water so as to attain 14 g/100 mL and stirred for 10 seconds, and the solution within 1 minute from preparation was used for administration. |
| Administration group B | The pharmaceutical composition of Example 22 was added to purified water so as to attain 14 g/100 mL and stirred for 10 seconds, and the solution within 1 minute from preparation was used for administration. |

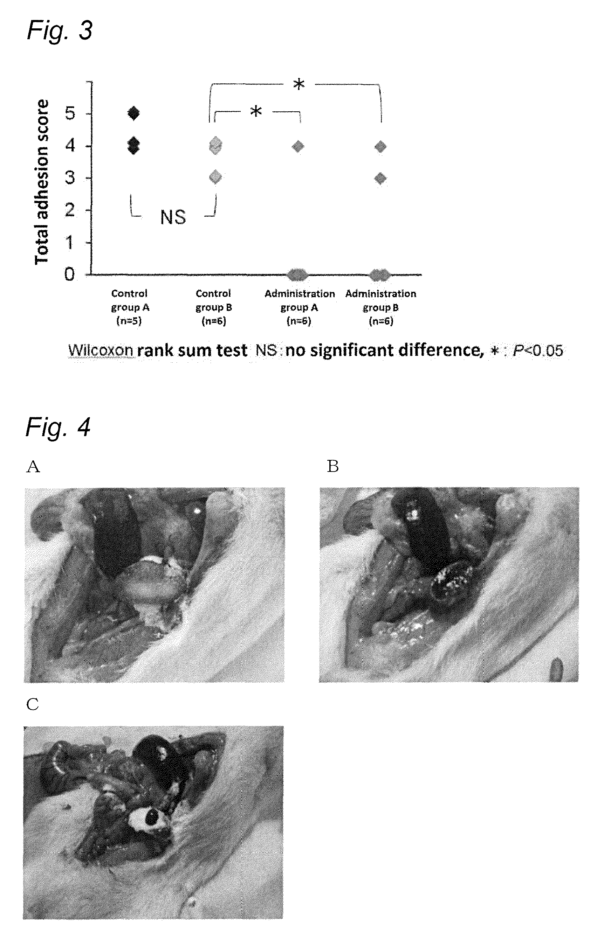

The obtained results are shown in FIG. 3. According to the results, all 6 subjects administered with the lactated Ringer's solution in the control group B developed adhesion, whereas adhesion was not developed in 5 subjects out of 6 subjects in administration group A, and adhesion was not observed in 4 subjects out of 6 subjects in administration group B. It is clear from the results presented above that use of the pharmaceutical composition of the present invention makes it possible to effectively inhibit adhesion of organs.

Test Example 10. Hemostatic effect 1

<Method A: Evaluation of hemostatic effect on non-continuous bleeding

Laparotomy was performed on male rats (Crlj :WI) (obtained from Charles River Laboratories Japan, Inc.) to expose the left kidney, then the left renal artery and vein were clamped, blood oozing therefrom was removed, 0.3 g of the powdery pharmaceutical composition of Example 57 was spread on the clamped site, and pressure was applied thereto for 1 minute with wet gauze that had been dampened with physiological saline. Thereafter, the clamp was removed, and presence or absence of bleeding was visually checked for 5 minutes. As a comparative example, sodium alginate power was spread, and measurement was carried out in the same manner.

<Method B: Evaluation of hemostatic effect on continuous bleeding>

A test was carried out in the same manner as in method A except that the clamp was removed before spreading the composition.

In method A, the powdery pharmaceutical composition of Example 57 was spread. The state reached 5 minutes after clamp removal is shown in FIG. 4A, the state after removing the gelled pharmaceutical composition of Example 57 is shown in FIG. 4B, and the state reached 5 minutes after clamp removal following spreading the comparative example (sodium alginate powder) is shown in FIG. 4C. As shown in FIG. 4, in the case where the powdery pharmaceutical composition of Example 57 was spread, no bleeding was visible at 5 minutes after clamp removal. Also, bleeding was not visible even when the gelled composition was removed. On the other hand, in the comparative example (in which sodium alginate powder was spread), overt bleeding continued even at 5 minutes after clamp removal, and thus no hemostatic effect was observed. Also, in method B, the powdery pharmaceutical composition of Example 57 showed a nearly identical hemostatic effect, but no hemostatic effect was observed with the sodium alginate powder of the comparative example.

Test Example 11. Hemostatic effect 2

The hemostatic effect was evaluated using male rats (Crlj:WI) (obtained from Charles River Laboratories Japan, Inc.). Specifically, the left renal artery and vein of a rat were clamped, and 1/3 of the kidney was excised. Next, oozing blood was removed, the test substances shown in Table 14 were administered to the excision site of the kidney. Then, about 2 mL of physiological saline was spread using a syringe equipped with a spray nozzle on the test substance administered to the excision site of the kidney, and left to stand for 1 minute. The clamp was then removed. For 5 minutes from clamp removal, the blood from the excision site of the kidney was absorbed into medical gauze, and the difference between the weights of the gauze before and after absorption was calculated as the amount of bleeding.

Table 14

|  | Test substance |
| --- | --- |
| Control group I | None |
| Control group II | Sodium alginate (Kimica Algin I-series I-1, viscosity of 1 w/v% aqueous solution at 20°C = 80 to 200 mPa·s, manufactured by Kimica Corporation); administered in an amount of $45.0 \pm 7.1$ mg |
| Administration group I | Powdery pharmaceutical composition containing 60.6 wt% of sodium alginate (Kimica Algin I-series I-1, viscosity of 1 w/v% aqueous solution at 20°C = 80 to 200 mPa·s, manufactured by Kimica Corporation), 3.0 wt% of macrogol 4000, 6.1 wt% of calcium hydrogenphosphate (manufactured by Wako Pure Chemical Industries, Ltd.), and 30.3 wt% of glucono-$\delta$-lactone (manufactured by Spectrum Chemical Mfg. Corp., USA) (prepared in the same manner as in Example 1) ; administered in an amount of $60.0 \pm 14.1$ mg |

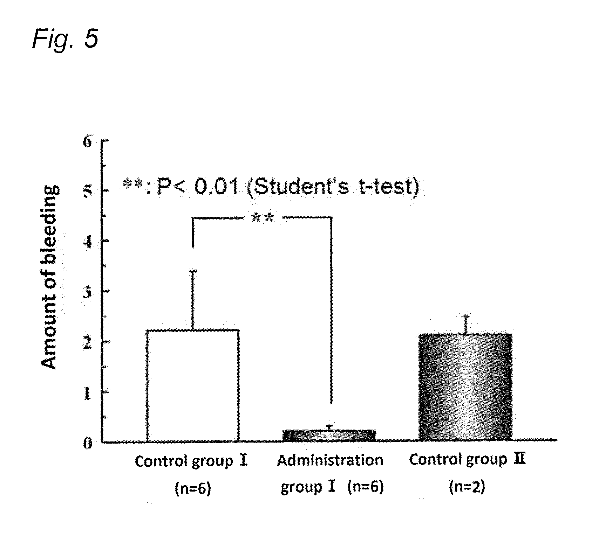

The obtained results are shown in FIG. 5. It was confirmed from the results that the amount of bleeding in administration group I was significantly smaller than in control groups I and II, and thus the pharmaceutical composition of the present invention has an excellent hemostatic effect.

**Claims**

1. A solid pharmaceutical composition comprising (A) a gelling agent, (B) a salt of a divalent metal and at least one selected from the group consisting of organic acids and inorganic acids, and (C) polyethylene glycol.

2. The pharmaceutical composition according to claim 1, further comprising (D) an organic acid or an alkali metal salt thereof.

3. The pharmaceutical composition according to claim 1 or 2, wherein the component (A) is alginic acid, a pharmaceutically acceptable alginic acid salt, or pectin.

4. The pharmaceutical composition according to any of claims 1 to 3, wherein the divalent metal in the component (B) is calcium.

5. The pharmaceutical composition according to any of claims 1 to 4, wherein the component (B) is at least one selected from the group consisting of dibasic calcium phosphate and calcium gluconate.

6. The pharmaceutical composition according to any of claims 1 to 5, wherein the component (C) has an average molecular weight of about 1000 to about 20000.

7. The pharmaceutical composition according to any of claims 1 to 6, wherein the component (C) is macrogol 4000.

8. The pharmaceutical composition according to any of claims 1 to 7, wherein the component (D) is gluconic acid or glucono-δ-lactone.

9. The pharmaceutical composition according to any of claims 1 to 8, comprising 3 to 50 parts by weight of the component (D) relative to 1 part by weight of the component (B).

10. The pharmaceutical composition according to any of claims 1 to 9, used as an adhesion preventing biomedical material.

11. The pharmaceutical composition according to any of claims 1 to 9, used as a hemostatic agent.

12. Use of a solid pharmaceutical composition comprising (A) a gelling agent, (B) a salt of a divalent metal and at least one selected from the group consisting of organic acids and inorganic acids, and (C) polyethylene glycol, for the manufacture of an adhesion preventing biomedical material.

13. Use of a solid pharmaceutical composition containing (A) a gelling agent, (B) a salt of a divalent metal and at least one selected from the group consisting of organic acids and inorganic acids, and (C) polyethylene glycol, for the manufacture of a hemostatic agent.

14. An adhesion preventing method comprising the steps of:

preparing a solution by mixing an aqueous solvent with a solid pharmaceutical composition containing (A) a gelling agent, (B) a salt of a divalent metal and at least one selected from the group consisting of organic acids and inorganic acids, and (C) polyethylene glycol; and
administering the solution to an affected part where adhesion prevention is required.

15. A hemostatic method comprising the step of administering a solid pharmaceutical composition containing (A) a gelling agent, (B) a salt of a divalent metal and at least one selected from the group consisting of organic acids and inorganic acids, and (C) polyethylene glycol to an affected part where hemostasis is required.

*Fig. 1*

*Fig. 2*

*Fig. 3*

Wilcoxon rank sum test NS: no significant difference, *: P<0.05

*Fig. 4*

A

B

C

*Fig. 5*

## INTERNATIONAL SEARCH REPORT

| International application No. |
|---|
| PCT/JP2012/080339 |

### A. CLASSIFICATION OF SUBJECT MATTER
*A61L15/16*(2006.01)i, *A61K31/734*(2006.01)i, *A61K45/00*(2006.01)i, *A61K47/04* (2006.01)i, *A61K47/12*(2006.01)i, *A61K47/22*(2006.01)i, *A61K47/34*(2006.01)i, *A61L27/00*(2006.01)i, *A61P7/04*(2006.01)i

According to International Patent Classification (IPC) or to both national classification and IPC

### B. FIELDS SEARCHED

Minimum documentation searched (classification system followed by classification symbols)
A61L15/16, A61K31/734, A61K45/00, A61K47/04, A61K47/12, A61K47/22, A61K47/34, A61L27/00, A61P7/04

Documentation searched other than minimum documentation to the extent that such documents are included in the fields searched

| Jitsuyo Shinan Koho | 1922-1996 | Jitsuyo Shinan Toroku Koho | 1996-2012 |
|---|---|---|---|
| Kokai Jitsuyo Shinan Koho | 1971-2012 | Toroku Jitsuyo Shinan Koho | 1994-2012 |

Electronic data base consulted during the international search (name of data base and, where practicable, search terms used)
MEDLINE/CAplus/EMBASE/BIOSIS(STN), JSTPlus/JMEDPlus/JST7580(JDreamII)

### C. DOCUMENTS CONSIDERED TO BE RELEVANT

| Category* | Citation of document, with indication, where appropriate, of the relevant passages | Relevant to claim No. |
|---|---|---|
| X/Y | JP 10-58318 A (Terumo Corp.), 07 April 1998 (07.04.1998), claims; paragraphs [0001], [0011], [0016] (Family: none) | 1,6,7/2-5, 8-13 |
| X/A | JP 2005-533534 A (Insense Ltd.), 10 November 2005 (10.11.2005), paragraphs [0031] to [0034], [0109] & WO 2003/090800 A1 & US 2006/0034816 A1 & EP 1358893 A1 | 1,3,4/2,5-13 |
| X/Y | JP 2003-531682 A (Fziomed, Inc.), 28 October 2003 (28.10.2003), claims; paragraphs [0036] to [0040], [0083] & WO 2001/082937 A1 & US 2002/0010150 A1 & EP 1292316 A | 1-4,6,10-13/ 5,7-9 |

| ☒ Further documents are listed in the continuation of Box C. | ☐ See patent family annex. |
|---|---|

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 December, 2012 (17.12.12) | 08 January, 2013 (08.01.13) |

| Name and mailing address of the ISA/ Japanese Patent Office | Authorized officer |
|---|---|
| Facsimile No. | Telephone No. |

Form PCT/ISA/210 (second sheet) (July 2009)

EP 2 783 704 A1

**INTERNATIONAL SEARCH REPORT**

International application No.

PCT/JP2012/080339

C (Continuation). DOCUMENTS CONSIDERED TO BE RELEVANT

| Category* | Citation of document, with indication, where appropriate, of the relevant passages | Relevant to claim No. |
|---|---|---|
| Y | JP 3-09761 A (Johnson & Johnson Patient Care, Inc.), 17 January 1991 (17.01.1991), page 3, upper right column to lower left column & GB 8818114 A | 1-13 |
| Y | JP 2010-537717 A (FMC Corp.), 09 December 2010 (09.12.2010), paragraph [0006] & WO 2009/032158 A2 & US 2011/0053886 A1 & EP 2192906 A | 1-13 |
| Y | JP 2002-522469 A (Incept L.L.C.), 23 July 2002 (23.07.2002), paragraph [0095] & WO 2000/009087 A1 & US 6514534 B1 & EP 1105094 A | 1-13 |
| A | WO 2010/050787 A2 (Genewel Co., Ltd.), 06 May 2010 (06.05.2010), claims & JP 2012-506903 A & EP 2351556 A2 & WO 2010/050787 A2 | 1-13 |
| A | JP 8-24325 A (Kuraray Co., Ltd.), 30 January 1996 (30.01.1996), entire text & WO 1995/031223 A1 & US 5658592 A & EP 0712635 A1 | 1-13 |

Form PCT/ISA/210 (continuation of second sheet) (July 2009)

31

**INTERNATIONAL SEARCH REPORT**

| International application No. |
| --- |
| PCT/JP2012/080339 |

| Box No. II Observations where certain claims were found unsearchable (Continuation of item 2 of first sheet) |
| --- |

This international search report has not been established in respect of certain claims under Article 17(2)(a) for the following reasons:

1. ☒ Claims Nos.: 14,15
   because they relate to subject matter not required to be searched by this Authority, namely:
   The inventions set forth in claims 14 and 15 pertain to therapeutic method for a human being and thus relate to a subject matter on which this International Searching Authority is not required to prepare an opinion.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III Observations where unity of invention is lacking (Continuation of item 3 of first sheet) |
| --- |

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.

PCT/JP2012/080339

Claims 1, 12 and 13 relate to a solid pharmaceutical composition which contains, as an active ingredient, a compound that is defined by a desired property of being "a gelling agent".

Although claims 1, 12 and 13 include every compound that has such a property, only a few compounds among the claimed compounds are disclosed within the meaning of PCT Article 5. Consequently, claims 1, 12 and 13 are not considered to be fully supported within the meaning of PCT Article 6.

Meanwhile, with respect to the "gelling agent", since the scope of compounds having such a property cannot be specified even by taking the common technical knowledge at the time of filing into consideration, claims 1, 12 and 13 also fail to satisfy the requirement of clearness prescribed under PCT Article 6.

Consequently, the international search has been carried out on a solid pharmaceutical composition which contains, as an active ingredient, a compound such as alginic acid that is specifically set forth in paragraph [0016] of the description.

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11253547 A
- JP 2003153999 A
- JP 7136240 A

**Non-patent literature cited in the description**

- The Japanese Pharmacopoeia